# EUROPEAN PATENT APPLICATION

(11) **EP 1 097 997 A1**
(43) Date of publication of application: **09.05.2001**
(21) Application number: 99402723.3
(22) Date of filing: 03.11.1999
(51) Int. Cl.: C12N 15/57, C12N 9/48, C07K 16/40, A61K 38/48

(54) **Human carnosinase, its isolation and uses**

(71) Applicant: SANOFI-SYNTHELABO, 75013 Paris (FR)
(72) Inventor: Saudek, Vladimir, 67300 Schiltigheim (FR); Smirnova-Robert, Tatiana, 92330 Sceaux (FR); Teufel, Michael, 77767 Appenweier (DE)
(74) Representative: Thouret-Lemaitre, Elisabeth

(57) **Abstract**

HC polypeptides and polynucleotides and methods for producing such polypeptides by recombinant techniques are disclosed. Also disclosed are methods for utilizing HC polypeptides and polynucleotides in the design of protocols for the treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock., epilepsia, among others, and diagnostic assays for such conditions.

SEQUENCE LISTING

<110> SANOFI-SYNTHELABO

<120> Human carnosinase, its isolation and uses.

<130> SANSYL010

<160> 2

<170> PatentIn Ver. 2.1

<210> 1
<211> 1524
<212> DNA
<213> Homo sapiens

<220>
<221> CDS
<222> (1)..(1524)

<400> 1
atg gat ccc aaa ctc ggg aga atg gct gcg tcc ctg ctg gct gtg ctg     48
ctg ctg ctg ctg ctg gag cgc ggc atg ttc tcc tca ccc tcc ccg ccc     96
ccg gcg ctg tta gag aaa gtc ttc cag tac att gac ctc cat cag gat    144
gaa ttt gtg cag acg ctg aag gag tgg gtg gcc atc gag agc gac tct    192
gtc cag cct gtg cct cgc ttc aga caa gag ctc ttc aga atg atg gcc    240
gtg gct gcg gac acg ctg cag cgc ctg ggg gcc cgt gtg gcc tcg gtg    288
gac atg ggt cct cag cag ctg ccc gat ggt cag agt ctt cca ata cct    336
ccc gtc atc ctg gcc gaa ctg ggg agc gat ccc acg aaa ggc acc gtg    384
tgc ttc tac ggc cac ttg gac gtg cag cct gct gac cgg ggc gat ggg    432
tgg ctc acg gac ccc tat gtg ctg acg gag gta gac ggg aaa ctt tat    480
gga cga gga gcg acc gac aac aaa ggc cct gtc ttg gct tgg atc aat    528
gct gtg agc gcc ttc aga gcc ctg gag caa gat ctt cct gtg aat atc     576
aaa ttc atc att gag ggg atg gaa gag gct ggc tct gtt gcc ctg gag     624
gaa ctt gtg gaa aaa gaa aag gac cga ttc ttc tct ggt gtg gac tac     672
att gta att tca gat aac ctg tgg atc agc caa agg aag cca gca atc     720
act tat gga acc cgg ggg aac agc tac ttc atg gtg gag gtg aaa tgc     768
aga gac cag gat ttt cac tca gga acc ttt ggt ggc atc ctt cat gaa     816
cca atg gct gat ctg gtt gct ctt ctc ggt agc ctg gta gac tcg tct     864
ggt cat atc ctg gtc cct gga atc tat gat gaa gtg gtt cct ctt aca     912
gaa gag gaa ata aat aca tac aaa gcc atc cat cta gac cta gaa gaa     960
tac cgg aat agc agc cgg gtt gag aaa ttt ctg ttc gat act aag gag     1008
gag att cta atg cac ctc tgg agg tac cca tct ctt tct att cat ggg     1056
atc gag ggc gcg ttt gat gag cct gga act aaa aca gtc ata cct ggc     1104
cga gtt ata gga aaa ttt tca atc cgt cta gtc cct cac atg aat gtg     1152
tct gcg gtg gaa aaa cag gtg aca cga cat ctt gaa gat gtg ttc tcc     1200
aaa aga aat agt tcc aac aag atg gtt gtt tcc atg act cta gga cta     1248
cac ccg tgg att gca aat att gat gac acc cag tat ctc gca gca aaa     1296
aga gcg atc aga aca gtg ttt gga aca gaa cca gat atg atc cgg gat     1344
gga tcc acc att cca att gcc aaa atg ttc cag gag atc gtc cac aag     1392
agc gtg gtg cta att ccg ttg gga gct gtt gat gat gga gaa cat tcg     1440
cag aat gag aaa atc aac agg tgg aac tac ata gag gga acc aaa tta     1488
ttt gct gcc ttt ttc tta gag atg gcc cag ctc cat                     1524


<210> 2
<211> 508
<212> PRT
<213> Homo sapiens

<400> 2

## Description

The invention relates to newly identified polynucleotides, polypeptides encoded by them and to the use of such polynucleotides and polypeptides, and to their production. More particularly, the polynucleotides and polypeptides of the present invention relate to human carnosinase, hereinafter referred to as HC, and its use. The invention also relates to inhibiting or activating the action of such polynucleotides and polypeptides.

Carnosine and related dipeptides such as anserine are naturally-occurring histidine-containing compounds. They are found in several tissues, most notably in muscle, where they represent an appreciable fraction of the total water-soluble nitrogen-containing compounds. The biological roles of these dipeptides are conjectural but they are believed to act as cytosolic buffering agents. Numerous studies have demonstrated, both at the tissue and organelle levels, that they possess strong and specific antioxidant properties. Carnosine and related dipeptides have been shown to prevent peroxidation of model membrane systems, leading to the suggestion that they represent water-soluble counterparts of lipid-soluble antioxidants, such as alpha-tocopherol, in protecting cell membranes from oxidative damage. Other roles ascribed to these dipeptides include actions as neurotransmitters, modulation of enzymatic activities and chelation of heavy metals. Many therapeutic actions of carnosine and histidine-containing dipeptides have been suggested, including antihypertensive effects, actions as immunomodulating agents, wound healing and antineoplastic effects *(Mol Aspects Med* 1992;13(5):379-444).

Carnosine and anserine are hydrolyzed in serum by the enzyme carnosinase. Carnosinase from human plasma has been purified *(Clin Chim Acta* 1991 Feb 15;196(2-3):193-205). Human serum carnosinase is found to be a glycoprotein with an isoelectric point of 4.4 and a subunit molecular weight of 75,000; the active enzyme being a dimer and the two subunits being connected by one or more disulfide bonds. The enzyme is especially active in hydrolyzing carnosine and anserine, preferring dipeptides which contain histidine in the C-terminal position. Brain contains a high amount of carnosinase. The physiological function of this enzyme seems to be the hydrolysis of homocarnosine in the brain and the splitting of carnosine and anserine in the blood stream.

Several reports describe variable phenotypes for a serum carnosinase deficiency, which range from normal to severe psychomotor retardation, hypotonia, and myoclonic seizures in the first year of life. A terminal deletion of chromosome 18 with breakpoint at q21.3 has been reported in the case of a 30-month-old girl with hypotonia, developmental delays, and tremor. Serum carnosinase activity of this girl was found to be extremely low. These findings suggest that the locus for carnosinase resides on the distal long arm of chromosome 18 *(Pediatric Res.* 1997 Feb;41(2):210-3).

Two unrelated children with a progressive neurologic disorder characterized by severe mental defect and myoclonic seizures have been described *(Lancet* I: 1229-1230, 1968). Both excreted carnosine in the urine, even when any source of the dipeptide is excluded from the diet. Both had unusually high concentrations of homocarnosine in the cerebrospinal fluid. It was suggested that one, and perhaps both, presented a defect in carnosinase activity, this enzyme being almost absent in the two patients. Autopsy on one of the boys (7-year old), whose parents had a low carnosinase activity, showed severe axonal degeneration, numerous 'spheroids' in the gray matter, demyelinization, fibrosis, and loss of Purkinje fibers *(Neurology* 22: 644-654, 1972).

Measurement of Human Serum Carnosinase may be useful in the assessment of patients with acute stroke with respect to diagnosis and prediction of clinical outcome *(Stroke* 1996 Nov;27(11):2064-8).

Serum carnosinase activity is shown to be altered in patients with neurological disorders. Reduced serum carnosinase activity has been observed in patients with Parkinson's disease and multiple sclerosis compared with the control group. Carnosinase activity, 5-10% of that found in serum, was detected in cerebrospinal fluid (CSF) samples *(Clin Chim Acta* 1994 Feb;225(1):57-64). The cause of reduced serum carnosinase activity in central nervous system disorders remains unclear.

An interesting finding suggests that in addition to glutamate decarboxylation, a second metabolic reaction for the formation of free GABA exists in specific neuronal tracts of the human CNS where GABA is released from homocarnosine by the action of serum carnosinase *(Brain Res. Bull.* 1994;33(4):379-85).

This indicates that human carnosinase has an established, proven history as therapeutic target and that it can play a role in preventing, ameliorating or correcting dysfunctions or diseases, including, but not limited to, cognitive disorders, developmental abnormalities and fetal deficiencies, neurodegenerative disorders such as amyotrophic lateral sclerosis, Parkinson's disease, schizophrenia, abnormal mental states, ischemic shock, epilepsia.

In one aspect, the invention relates to HC polypeptides and polynucleotides, and to recombinant materials and methods for their production. Another aspect of the invention relates to methods for using such HC polypeptides and polynucleotides, for example in the treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.

In still another aspect, the invention relates to methods to identify HC agonists and antagonists using the materials provided by the invention, and treating conditions associated with HC. Yet another aspect of the invention relates to diagnostic assays for detecting diseases associated with inappropriate HC activity or levels.

Figure 1 is an histogram showing the relative abundance of the messenger RNA of HC in different human tissues.

Figure 2 is an histogram of the micro-fluorescent measurement of activity of HC in transfected CHO cells.

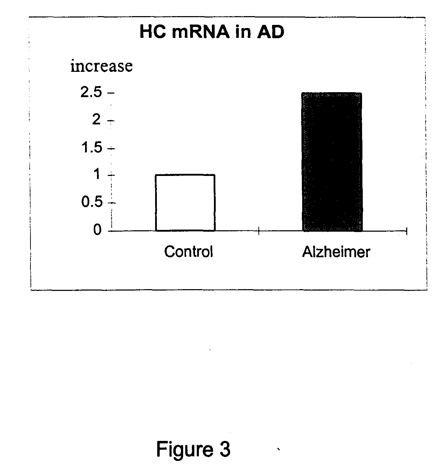

Figure 3 is an histogram showing the abundance of mRNA level of HC in patients with Alzheimer's disease (AD) compared with control patients.

The following definitions are provided to facilitate understanding of certain terms used frequently herein.

"HC" or "HC polypeptide" refers generally to a polypeptide having the amino acid sequence set forth in SEQ ID NO:2 or an allelic variant thereof, as well as the variants as described further below.

"HC activity" or "HC polypeptide activity" or "biological activity of HC" or "biological activity of HC polypeptide" refers to the metabolic or physiologic function of said HC including similar activities or improved activities, or these activities with decreased undesirable side-effects. Also included are antigenic and immunogenic activities of said HC.

"HC gene" refers to a polynucleotide having the nucleotide sequence set forth in SEQ ID NO:1 or allelic variants thereof and/or their complements as described in the following specification.

"Antibodies" as used herein includes polyclonal and monoclonal antibodies, chimeric, single chain, and humanized antibodies (an antibody - mouse or any other-with some specific parts changed to human homologous regions), as well as Fab fragments, including the products of a Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein. For example, recombinant DNA molecules contained in a vector are considered isolated for the purposes of the present invention. Further examples of isolated nucleic acid molecules include recombinant DNA molecules maintained in heterologous host cells or purified DNA molecules in solution. Isolated RNA molecules include *in vivo* or *in vitro* RNA transcripts of the DNA molecules of the present invention. Isolated nucleic acid molecules according to the invention further include such molecules produced synthetically.

"Polynucleotide" generally refers to any polyribonucleotide or polydeoxyribonucleotide, which may be unmodified RNA or DNA or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications have been made to DNA and RNA; thus, "polynucleotide" embraces chemically, enzymatically or metabolically modified forms of polynucleotides as typically found in nature, as well as the chemical forms of DNA and RNA characteristic of viruses and cells. Thus, "polynucleotide" also encompasses variants of the nucleic acid molecules of the invention which encode portions, analogs or derivatives of HC. Variants may occur naturally, such as natural allelic variants. By an "allelic variant" is intended one of several alternate forms of a gene occupying a given locus on a chromosome of an organism. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, Proteins - Structure and Molecular Properties, 2nd Ed., T. E. Creighton, W. H. Freeman and Company, New York, 1993 and Wold, F., Post-translational Protein Modifications: Perspectives and Prospects, p. 1-12 in Post-translational Covalent Modification of Proteins, B. C. Johnson, Ed., Academic Press, New York, 1983; Seifter *et al.,* "Analysis for protein modifications and non-protein cofactors", *Meth. Enzymol.* (1990) 182:626-646 and Rattan *et al.,* "Protein Synthesis: Post-translational Modifications and Aging", *Ann NY Acad Sci* (1992) 663:48-62.

"Variant" as the term is used herein, represents a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"Identity" is a measure of the identity of nucleotide sequences or amino acid sequences. In general, the sequences are aligned so that the highest order match is obtained. "Identity" *per se* has an art-recognized meaning and can be calculated using published techniques. See, e.g.: Computational Molecular Biology, Lesk, A. M., ed., Oxford University Press, New York, 1988; Biocomputing : Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991. While there exists a number of methods to measure identity between two polynucleotide or polypeptide sequences, the term "identity" is well known to skilled artisans (Carillo, H., and Lipton, D., *SIAM J Applied Math* (1988) 48:1073). Methods commonly employed to determine identity or similarity between two sequences include, but are not limited to, those disclosed in Guide to Huge Computers, Martin J. Bishop, ed., Academic Press, San Diego, 1994, and Carillo, H., and Lipton, D., *SIAM J Applied Math* (1988) 48:1073. Methods to determine identity and similarity are codified in computer programs. Preferred computer program methods to determine identity and similarity between two sequences include, but are not limited to, GCG program package (Devereux, J., *et al., Nucleic Acids Research* (1984) 12(1):387), BLASTP, BLASTN, FASTA (Atschul, S.F. *et al., J. Molec. Biol.* (1990) 215:403).

As an illustration, by a polynucleotide having a nucleotide sequence having at least, for example, 95% "identity" to a reference nucleotide sequence is intended a polynucleotide in which the nucleotide sequence is identical to the reference sequence except that the polynucleotide sequence may include up to five point mutations per each 100 nucleotides of the reference nucleotide sequence. In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence.

Similarly, by a polypeptide having an amino acid sequence having at least, for example, 95% "identity" to a reference amino acid sequence is intended a polypeptide having an amino acid sequence identical to the reference sequence except that the polypeptide sequence may include up to five amino acid alterations per each 100 amino acids of the reference amino acid sequence. In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

In one aspect, the present invention relates to HC polypeptides. The HC polypeptides of the invention include the polypeptide of SEQ ID NO:2; as well as polypeptides comprising the amino acid sequence of SEQ ID NO: 2; and polypeptides comprising the amino acid sequences which have at least 80% identity to that of SEQ ID NO:2 over its entire length, and still more preferably at least 90% identity, and even still more preferably at least 95% identity to SEQ ID NO: 2. Furthermore, those with at least 97-99% identity are highly preferred. Also included within HC polypeptides are polypeptides having the amino acid sequences which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% identity are highly preferred. Preferably HC polypeptides exhibit at least one biological activity of HC. It will be recognized that some amino acids of the HC polypeptides can be varied without significant effect on the structure or function of the protein. If such differences are contemplated, it should be remembered that there will be critical areas on the protein which determine activity.

Thus, the invention further provides variants of the HC polypeptides which show substantial HC polypeptide activity or which include regions of HC polypeptide such as the protein portions discussed below. Such mutants include HC polypeptides in which deletions, insertions, type substitutions, inversions and/or repeats have occurred. Guidance concerning amino acid changes which are likely to be phenotypically silent can be found in Bowie, J. U. et al, "Deciphering the Message in Protein Sequences : Tolerance to Amino Acid Substitutions", *Science* 247 : 1306-1310 (1990).

Thus, the fragment, derivative or analog of the polypeptide of SEQ ID NO:2, or that encoded by the deposited cDNA as discussed below, may be :
- one in which one or more of the amino acid residues are substituted with a conserved or non-conserved amino acid residue, preferably a conserved amino acid residue, and such substituted amino acid residue may or may not be one encoded by the genetic code, or
- one in which one or more of the amino acid residues includes a substituent group, or
- one in which the mature polypeptide is fused with another compound, such as a compound for increasing the half-life of the polypeptide (for example, polyethylene glycol), or
- one in which additional amino acids are fused to the mature polypeptide, such as the IgG Fc fusion region peptide or leader or secretory sequence which is employed for purification of the mature polypeptide or a pro-protein sequence. Such fragments, derivatives and analogs are deemed to be within the scope of the invention.

Of particular interest are substitutions of charged amino acids with another charged amino acid and with neutral or negatively charged amino acids. The latter results in proteins with reduced positive charge to improve the characteristics of the HC polypeptide, for example for prevention of aggregation.

As indicated, changes are preferably of a minor nature, such as conservative amino acid substitutions that do not significantly affect the activity of the polypeptide.

Examples of amino acids which can be used for substitution are given hereafter.

| | |
|---|---|
| Aromatic | Phenylalanine |
| | Tryptophan |
| | Tyrosine |
| Hydrophobic | Leucine |
| | Isoleucine |
| | Valine |
| Polar | Glutamine |
| | Asparagine |
| Basic | Arginine |
| | Lysine |
| | Histidine |
| Acidic | Aspartic acid |
| | Glutamic acid |
| Small | Alanine |
| | Serine |
| | Threonine |
| | Methionine |
| | Glycine |

Generally, the number of amino acid substitutions will not be more than 50.

Amino acids in the HC polypeptide of the present invention that are essential for function can be identified by methods known in the art, such as site-directed mutagenesis or alanine-scanning mutagenesis (Cunnigham and Wells, *Science* 244 : 1081-1085 (1989)). The latter procedure introduces single alanine mutations at every residue in the molecule. The resulting mutant molecules are then tested for biological activity such as receptor binding or *in vitro* proliferative activity. Sites that are critical for ligand-receptor binding can also be determined by structural analysis, e.g. crystallization, nuclear magnetic resonance or photoaffinity labeling (Smith et al., *J*. *Mol. Biol.* 224 : 899-904 (1992); de Vos et al., *Science* 255 : 306-312 (1992)).

As to the selection of peptides or polypeptides bearing an antigenic epitope, it is well known that relatively short synthetic sequences that mimic part of a protein sequence are routinely capable of eliciting an antiserum that reacts with the partially mimicked protein. See for instance Sutcliffe, J. G., Shinnick, T. M., Green, N. and Learner, R. A., Antibodies that React with Predetermined Sites on Proteins, *Science* 219 : 660-666 (1983). Peptides capable of eliciting protein-reactive sera are frequently represented in the primary sequence of a protein, can be characterized by a set of simple chemical rules, and are confined neither to immunodominant regions of intact proteins (i.e. immunogenic epitopes) nor to the amino or carboxyl terminals.

Antigenic epitope-bearing peptides and polypeptides of the invention are therefore useful to raise antibodies, including monoclonal antibodies, that bind specifically to a polypeptide of the invention. Antigenic epitope-bearing peptides and polypeptides of the invention preferably contain a sequence of at least seven, more preferably at least nine and most preferably between about 15 to about 50 amino acids contained within the amino acid sequence of a polypeptide according to the invention.

For example, antigenic polypeptides or peptides that can be used to generate specific HC antibodies comprise : a polypeptide comprising amino acid residues from about 255 to about 272 of SEQ ID NO:2, a polypeptide comprising amino acid residues from about 475 to about 490 of SEQ ID NO:2. As indicated above, it is believed that the above polypeptide fragments are antigenic regions of the HC polypeptide as determined using Protean (version 3.16) from DNASTAR. In particular, polypeptides comprising amino acid residues from 255 to 272 and amino acid residues from 475 to 490 of SEQ ID NO:2 were successfully used to raise polyclonal antibodies in rabbits, which antibodies are able to recognize HC in homogenates of human brain samples.

The epitope-bearing peptides and polypeptides of the invention can be prepared by any conventional means, for instance by the method described in document US Patent N° 4,631,211.

HC polypeptides of the present invention and the epitope-bearing fragments thereof can be combined with parts of the constant domain of immunoglobulins (lgG), resulting in chimeric polypeptides. These fusion proteins facilitate the purification and generally show an increased half life *in vivo.* See for instance Traunecker et al., *Nature* 331 : 84-86 (1988). Fusion proteins that have a disulfide-linked dimeric structure due to the lgG part can also be more efficient in binding and neutralizing other molecules than the monomeric HC protein or protein fragment thereof (Fountoulakis et al., *J. Biochem.* 270 : 3958-3964 (1995)).

The HC polypeptides may be in the form of the "mature" protein or may be a part of a larger protein such as a fusion protein. It is often advantageous to include an additional amino acid sequence which contains secretory or leader sequences, pro-sequences, sequences which aid in purification such as multiple histidine residues, or an additional sequence for stability during recombinant production.

Fragments of the HC polypeptides or variants, mutated forms, derivatives thereof as here above mentioned are also included in the invention. By a fragment is meant a polypeptide having an amino acid sequence that entirely is the same as part, but not all, of the amino acid sequence of the aforementioned HC polypeptides. As with HC polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of HC polypeptide shown in SEQ ID NO:1. In this context "about" includes the particularly recited ranges larger or smaller by several, for example 5, 4, 3, 2 or 1 amino acid at either extreme or at both extremes.

Preferred fragments include, for example, truncation polypeptides having the amino acid sequence of HC polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, tum and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate HC activity, including those with a similar activity or an improved activity, or with a decreased undesirable activity. Also included are those that are antigenic or immunogenic in an animal, especially in a human.

Preferably, all of these polypeptide fragments retain the biological activity of HC, including antigenic activity.

The HC polypeptides of the invention can be prepared in any suitable manner. Such polypeptides include isolated naturally occurring polypeptides, recombinantly produced polypeptides, or polypeptides produced by a combination of these methods. Means for preparing such polypeptides are well understood in the art.

In another embodiment, the HC polypeptides of the invention can be prepared by chemical synthesis, for example by using non natural and/or modified amino acids. Thus, it can be advantageous to use non natural amino acids, for example D-amino acids, to enhance life time of the polypeptides, or analogs of amino acids, for example sulfurized forms of amino acids.

Methods for preparing such polypeptides are known in the art.

For example, chemical synthesis of the polypeptides of the invention can be carried out by using a solid phase process. In such a method, the alpha-amino moiety of an amino acid is protected by using conventional protective groups (e.g. BOC or FMOC) and the thus protected amino acid is fixed onto an inert solid support by its C-terminal region. At the end of this step, the protective group is removed and a second amino acid, optionally protected in a similar manner, is fixed. The N-terminal protective groups are removed after each amino acid is fixed, protective groups possibly present on the lateral chains being conserved. When the peptide chain is synthesized, the peptide is cleaved from the support and the remaining protective groups are removed. Coupling can be carried out by using N,N'-diisopropyl-carbodiimine (DIC), among others, in a solvent of DMF or DCM. This solid phase synthesis is described for example in Stewart J. M. et al., Solid Phase Peptides Synthesis. Pierce Chem. Company, Rockford, 111, 2^{nd} Ed., (1984).

Other polypeptides according to the invention comprise the amino acid sequence of the HC polypeptide which possesses the complete amino acid sequence encoded by the cDNA clone contained in ATCC deposit number PTA-541 as discussed below, as well as polypeptides which comprise the amino acid sequence of the mature HC polypeptide which possesses the complete amino acid sequence encoded by the cDNA clone contained in ATCC deposit number PTA-541.

In another aspect, the invention provides a peptide or polypeptide comprising an epitope-bearing portion of a polypeptide of the invention. The epitope of this polypeptide portion is an immunogenic or antigenic epitope of a polypeptide described in the present invention. An immunogenic epitope is defined as a part of a protein that elicits an antibody response when the whole protein is the immunogen. On the other hand, a region of a protein molecule to which an antibody can bind is defined as an antigenic epitope.

Another aspect of the invention relates to HC polynucleotides.

Unless otherwise indicated, all nucleotide sequences determined by sequencing a DNA molecule herein have been determined by using an automated DNA sequencer, and all amino acid sequences of polypeptides encoded by DNA sequences determined herein have been predicted by translation of a DNA sequence determined as above. Therefore, as is known in the art for any DNA sequence determined by this approach, any nucleotide sequence may contain some errors. Nucleotide sequences determined by automation are typically at least about 95 % identical to about 99.9 % identical to the actual nucleotide sequence of the sequenced DNA molecule.

HC polynucleotides include isolated polynucleotides which encode the HC polypeptides and fragments, and polynucleotides closely related thereto. More specifically, HC polynucleotides of the invention include a polynucleotide comprising the nucleotide sequence set forth in SEQ ID NO:1 encoding a HC polypeptide of SEQ ID NO: 2, and a polynucleotide having the particular sequence of SEQ ID NO:1. HC polynucleotides of the invention further include a polynucleotide comprising a nucleotide sequence that has at least 80% identity to a nucleotide sequence encoding the HC polypeptide of SEQ ID NO:2 over its entire length, and a polynucleotide that is at least 80% identical to that having SEQ ID NO:1 over its entire length. In this regard, polynucleotides at least 90% identical are particularly preferred, and those with at least 95% identity are especially preferred. Furthermore, those with at least 97% identity are highly preferred and those with at least 98-99% identity are most highly preferred, with at least 99% identity being the most preferred. Also included under HC polynucleotide is a nucleotide sequence which has sufficient identity to the nucleotide sequence contained in SEQ ID NO:1 or contained in the cDNA insert of the plasmid deposited with the ATCC Deposit number PTA-541 discussed below to hybridize under conditions useable for amplification or for use as a probe or marker. Moreover, HC polynucleotides include a nucleotide sequence having at least 80% identity to a nucleotide sequence encoding the HC polypeptide, either in a mature form or not, expressed by the cDNA insert deposited at the ATCC with Deposit Number PTA-541 as discussed below, and a nucleotide sequence comprising at least 15 contiguous nucleotides of such cDNA insert. In this regard, polynucleotides at least 90% identical are particularly preferred, and those with at least 95% identity are especially preferred. Furthermore, those with at least 97% identity are highly preferred and those with at least 98-99% identity are most highly preferred, those with at least 99% identity being the most preferred. The invention also provides polynucleotides which are complementary to all HC polynucleotides according to the invention.

A deposit containing a human HC cDNA has been deposited with the American Type Culture Collection (ATCC), 10801 University Blvd, Manassas, VA 20110-2209, USA, on August 17, 1999, and assigned ATCC Deposit Number PTA-541. The deposited material (clone) is pCDNA3 that further contains the full length cDNA of HC, referred to as Human plasmid SGC-115 upon deposit. The nucleotide sequence of the polynucleotide contained in the deposited material, as well as the amino acid sequence of the polypeptide encoded thereby, are controlling in the event of any conflict with any description of sequences herein.

The deposit has been made under the terms of the Budapest Treaty on the international recognition of the deposit of micro-organisms for purposes of patent procedure.

HC of the invention is structurally related to other proteins of the Novel protease-like protein family, as shown by the results of sequencing the cDNA of SEQ ID NO:1 encoding HC. The cDNA sequence set forth in SEQ ID NO:1 contains an open reading frame (nucleotide numbers 1 to 1524) encoding a polypeptide of about 508 amino acids residues of SEQ ID NO:2, with a predicted leader sequence of about 28 amino acid residues and a deduced molecular weight of about 58.6 KDa. Amino acid sequence set forth in SEQ ID NO:2 has about 45 % identity (using Megalign with the Clustal algorithm from DNASTAR) in 470 amino acid residues with a yeast hypothetical 52.9 KD protein. Nucleotide sequence set forth in SEQ ID NO:1 has about 59.6% identity (Megalign with the Clustal algorithm from DNASTAR) in 1256 nucleotide residues with the yeast hypothetical protein 52.9 KD (CAB07646).

The polynucleotides of the present invention encoding HC may be obtained using standard cloning and screening, from a cDNA library derived from mRNA in cells of human brain using the expressed sequence tag (EST) analysis (Adams, M. D., *et al. Science* (1991) 252:1651-1656; Adams, M.. *et al., Nature,* (1992) 355:632-634; Adams, M. D., et *al., Nature* (1995) 377 Supp:3-174). Polynucleotides of the invention can also be obtained from natural sources such as genomic DNA libraries or can be synthesized using well known and commercially available techniques.

The nucleotide sequence encoding HC polypeptide of SEQ ID NO:2 may be identical to the polypeptide encoding sequence contained in SEQ ID NO:1, or it may be a different sequence, which as a result of the redundancy (degeneracy) of the genetic code, also encodes the polypeptide of SEQ ID NO:2.

As indicated, the invention also provides the mature form of the HC polypeptide of the present invention. According to the signal hypothesis, proteins secreted by mammalian cells have a signal or secretory leader sequence which is cleaved from the mature protein once export of the growing protein chain across the rough endoplasmic reticulum has been initiated. Most mammalian cells and even insect cells cleave secreted proteins with the same specificity. However, in some cases, cleavage of a secreted protein is not entirely uniform, which results in two or more mature species on the protein. Further, it has long been known that the cleavage specificity of a secreted protein is ultimately determined by the primary structure of the complete protein, i.e. its amino acid sequence. Therefore, the invention provides a nucleotide sequence encoding the mature HC polypeptide having the amino acid sequence encoded by the cDNA clone contained in the host identified as ATCC Deposit PTA-541. By the mature HC polypeptide having the amino acid sequence encoded by the cDNA clone contained in the host identified as ATCC Deposit PTA-541 is meant the mature form of HC polypeptide produced by expression in a mammalian cell of the complete open reading frame encoded by the human DNA sequence of the clone contained in the vector in the deposited host. As indicated, the mature HC having the amino acid sequence encoded by the cDNA clone contained in ATCC Deposit PTA-541 may or may not differ from the predicted "mature" HC protein represented in SEQ ID NO:2, depending on the accuracy of the predicted cleavage site based on computer analysis.

Methods for predicting whether a protein has a secretory leader as well as the cleavage site of this leader sequence are available. For instance, the methods of Mc Geoch *(Virus Res.* 3:271-286 (1985)) and von Heinje *(Nucleic Acids Res.* 14:4683-4690 (1986)) can be used.

When the polynucleotides of the invention are used for the recombinant production of HC polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro-protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc. Natl. Acad. Sci. USA* (1989) 86:821-824, or is an HA or V5 epitope tag. The polynucleotide may also contain non-coding 5' and 3' sequences, such as transcribed, non-translated sequences, splicing and polyadenylation signals, ribosome binding sites and sequences that stabilize mRNA.

Further preferred embodiments are polynucleotides encoding HC variants comprising the amino acid sequence HC polypeptide of SEQ ID NO:2 in which several, for example 5-10, 1-5, 1-3, 1-2 or 1 amino acid residues are substituted, deleted or added, in any combination.

The invention is further directed to fragments of the isolated nucleic acid molecules described herein. By a fragment of an isolated nucleic acid molecule having the nucleotide sequence of the deposited cDNA or the nucleotide molecule having the nucleotide sequence shown in SEQ ID NO:1 is intended fragments of at least about 15 nucleotides, and preferably of at least about 20 nucleotides, more preferably of at least about 30 nucleotides, even more preferably of at least about 40 nucleotides, which are useful as diagnostic probes and primers. By a fragment of at least 20 nucleotides in length, for example, are intended a fragment which includes 20 or more contiguous bases from the nucleotide sequence of the deposited cDNA or from the sequence shown in SEQ ID NO:1.

In a first aspect, preferred nucleic acid fragments according to the invention comprise those which can be used as nucleic probes.

For example, a nucleic probe according to the invention comprises the nucleotide sequence located between nucleotide number 1 and nucleotide number 500 of the sequence set forth in SEQ ID NO:1.

Another nucleic probe according to the invention comprises the nucleotide sequence located between nucleotide number 501 and nucleotide number 1524 of the sequence set forth in SEQ ID NO:1.

In a second aspect, preferred nucleic acid fragments according to the invention comprise a nucleotide sequence encoding for an antigenic region of HC.

For example, preferred nucleic acid fragments according to the invention comprise nucleic acid molecules which encode a polypeptide comprising amino acid residues from about 256 to about 272 of the amino acid sequence shown in SEQ ID NO:2, nucleic acid molecules which encode a polypeptide comprising amino acid residues from about 475 to about 490 of the amino acid sequence of SEQ ID NO:2.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 95% and preferably at least 97% identity between the sequences.

Polynucleotides of the invention, which are identical or sufficiently identical to a nucleotide sequence contained in SEQ ID NO:1 or a fragment thereof, may be used as hybridization probes for cDNA and genomic DNA, to isolate full-length cDNAs and genomic clones encoding HC polypeptide and to isolate cDNA and genomic clones of other genes that have a high sequence similarity to the HC gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

In one embodiment, to obtain a polynucleotide encoding HC polypeptide comprises the steps of screening an appropriate library under stringent hybridization conditions with a labeled probe having the SEQ ID NO: 1 or a fragment thereof; and isolating full-length cDNA and genomic clones containing said polynucleotide sequence. Such hybridization techniques are well known to those of skill in the art. Stringent hybridization conditions are as defined above or alternatively conditions under overnight incubation at 42°C in a solution comprising : 50% formamide, 5xSSC (150 mM NaCI, 15 mM trisodium citrate), 50 mM sodium phosphate (pH 7.6), 5x Denhardt's solution, 10 % dextran sulfate, and 20 microgram/ml denatured, sheared salmon sperm DNA, followed by washing the filters in 0.1xSSC at about 65°C.

The polynucleotides and polypeptides of the present invention may be employed as research reagents and materials for discovery of treatments and diagnostics of animal and human diseases.

The present invention also relates to recombinant vectors which comprise a polynucleotide or polynucleotides according to the invention and expression system capable of producing HC polypeptide when said expression system is present in a compatible host cell; host cells which are genetically engineered with vectors of the invention. The invention also relates to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et al., *Basic Methods in Molecular Biology* (1986) and Sambrook et al., *Molecular Cloning: a Laboratory Manual,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

The expression vectors will preferably include at least one selectable marker. Such markers include dihydrofolate reductase or neomycin resistance for eukaryotic cell culture and tetracycline or ampicillin resistance genes for culturing *E. coli and* other bacteria.

The DNA insert should preferably be linked to an appropriate promoter, such as the bacterial phage lambda PL and RP, T3 and T7, *E. coli lac, trp* and *tac* promoters, the eukaryotic SV40 early and late promoters, CMV immediate early promoters, eukaryotic promoters from retroviral LTRs, such as those of the Rous Sarcoma Virus (RSV) to name a few. Other suitable promoters are available for the skilled artisan.

The expression constructs will further contain sites for transcription initiation, termination and, in the transcribed region, a ribosome binding site for translation. The coding region of the mature transcripts expressed by the constructs will preferably include a translation initiating at the beginning and a termination codon (UAA, AGA or UAG) appropriately positioned at the end of the polypeptide to be translated.

Representative examples of appropriate hosts include bacterial cells, such as streptococci, staphylococci, *E. coli, Streptomyces, Salmonella typhimurium* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. Generally, any system or vector suitable to maintain, propagate or express polynucleotides, for producing a polypeptide in a host, may be used.

Among vectors preferred for use in bacteria are pGE70, pGE60 and pG-9, available form Qiagen, Phagescript vectors, pBS vectors, Bluescript vectors, pNH8A, pNH16a, pNH18A, pNH46A, available from Stratagene, ptrc99a, pKK223-3, pDR540, pRIT5 available from Pharmacia.

Among preferred eukaryotic vectors are pWLNEO, pSV2CAT, pOG44, pXT1 and pSG available from Stratagene, pSVK3, pBPV, pMSG and pSVL available from Pharmacia, pcDNA3, pcDNA3.1 and pTracer™ available from Invitrogene. Other suitable vectors will be readily apparent to the skilled in the art.

The appropriate nucleotide sequence may be inserted into an expression system by any of a variety of well-known and routine techniques, such as, for example, those set forth in Sambrook *et al., Molecular Cloning, a Laboratory Manual (supra).*

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the HC polypeptide is to be expressed for use in screening assays, generally, it is preferred that the polypeptide be produced at the surface of the cell. In this event, the cells may be harvested prior to use in the screening assay. If HC polypeptide is secreted into the medium, the medium can be recovered in order to recover and purify the polypeptide; if produced intracellularly, the cells must first be lysed before the polypeptide is recovered.

HC polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

The polypeptide may be expressed in a modified form, such as a fusion protein, and may include not only secretion signals, but also additional heterologous functional regions. For instance, a region of additional amino acids, particularly charged amino acids, may be added to the N-terminus of the polypeptide to improve stability and persistence in the host cell, during purification or during subsequent handling and storage. Also, peptide moieties may be added to the polypeptide to facilitate purification. Such regions may be removed prior to final preparation of the polypeptide. The addition of peptide moieties to polypeptides to engender secretion or excretion, to improve stability and to facilitate purification, among others, are familiar and routine techniques in the art. A preferred fusion protein comprises a heterologous region from immunoglobulin that is useful to solubilize proteins. For example, EP-A-0 464 533 discloses fusion proteins comprising various portions of constant region of immunoglobulin molecules together with another human protein or part thereof. In many cases, the Fc part in a fusion protein is advantageous for use in therapy and diagnosis. On the other hand, for some uses, it would be desirable to delete the Fc part after the fusion protein has been expressed, detected and purified. For instance, it is the case when Fc portion proves to be a hindrance to use in therapy and diagnosis, for example when the fusion protein is to be used as antigen for immunizations.

It is believed that mammals with certain diseases or susceptibility to certain diseases express significantly altered, enhanced or diminished levels of HC and mRNA encoding HC, when compared to a corresponding "standard" mammal, i.e. a mammal of the same species not having or not presenting a susceptibility to the disease. Further, it is believed that significantly altered, enhanced or diminished levels of HC can be detected in certain body tissues (e.g. bone marrow, cerebellum, brain, midbrain, spinal cord, nerve endings, retina, breast, pituitary, heart, lung, skeletal muscle, kidney, pancreas, intestine, stomach) from mammals with the disease or presenting a susceptibility to the disease when compared to tissues from mammals of the same species not having or not presenting a susceptibility to the disease. Thus, the invention provides a diagnostic method which involves assaying the expression level of the gene encoding a HC polypeptide of the present invention in mammalian cells or body fluid and comparing the gene expression level with a standard HC expression level, whereby an altered, enhanced or diminished expression level of the gene with respect to the standard is indicative of the disease or of a susceptibility to the disease. Preferred mammals include monkeys, apes, cats, dogs, cows, pigs, horses, rabbits and humans.

Diseases which can be diagnosed include but are not limited to Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock.

By "assaying the expression level of the gene encoding a HC polypeptide" is intended qualitatively or quantitatively measuring or estimating the level of HC or the level of the mRNA encoding HC in a first biological sample either directly (e.g. by determining or estimating absolute protein level or mRNA level) or relatively (e.g. by comparing with the HC polypeptide level or mRNA level in a second biological sample).

Preferably, the HC level or mRNA level in the first biological sample is measured or estimated and compared with a standard HC level or mRNA level, the standard being taken from a second biological sample obtained from an individual not having (nor presenting a susceptibility to) the disease. As will be appreciated, once a standard HC level or mRNA level is known, it can be used repeatedly as a standard for comparison.

By "biological sample" is intended any biological sample, either tissue sample or body fluid, obtained from an individual, cell line, tissue culture, or other source which may contain HC polypeptide or mRNA.

Total cellular RNA can be isolated from a biological sample using the single-step guanidinium-thiocyanate-phenol-chloroform method described in Chomczynski and Sacchi, *Anal. Biochem.* 162 : 156-159 (1987). Levels of mRNA encoding HC are then assayed using any appropriate method. These include Northern Blot analysis (Harada et al., *Cell* 63 : 303-312 (1990)), S1 nuclease mapping (Fujita et al., *Cell* 49 : 357-367 (1987)), the polymerase chain reaction (PCR), reverse transcription in combination with the polymerase chain reaction (RT-PCR) (Fujita et al., *Cell* 49 : 357-367 (1987)), and reverse transcription in combination with the ligase chain reaction (RT-LCR).

Assaying HC polypeptide levels in a biological sample can occur using antibody-based techniques. For example, HC expression in tissues can be studied with classical immunohistological methods (Jalkanen, M., et al., *J*. *Cell. Biol.* 101 : 976-985 (1985); Jalkanen, M., et al., *J*. *Cell. Biol. 105 : 3087-3096 (1987)).* Other antibody-based methods useful for detecting HC gene expression include immunoassays, such as the enzyme linked immunosorbent assay (ELISA) and the radioimmunoassay (RIA).

Suitable labels are known in the art and include enzyme labels, such as glucose oxidase, and radioisotopes, such as iodine (¹²⁵|, ¹²¹|), carbon (¹⁴C), sulfur (³⁵S), tritium (³H), indium (¹¹²|n), and technetium (^{99m}Tc), and fluorescent labels, such as fluorescein, rhodamine and biotin.

In addition, the invention provides a diagnostic assay for diagnosing or determining a susceptibility to, among others, Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock through detection of mutation in the HC gene by the methods described below.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled HC nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising HC nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example : M.Chee et al., Science, Vol 274, p 610-613 (1996)).

The nucleotide sequences of the present invention are also valuable for chromosome identification. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

The differences in the cDNA or genomic sequence between affected and unaffected individuals can also be determined. If a mutation is observed in some or all of the affected individuals but not in any normal individuals, then the mutation is likely to be the causative agent of the disease.

The polypeptides of the invention or their fragments or analogs thereof, or cells expressing them can also be used as immunogens to produce antibodies immunospecific for the HC polypeptides. The term "immunospecific" means that the antibodies have substantial greater affinity for the polypeptides of the invention than their affinity for other related polypeptides.

Antibodies generated against HC polypeptides can be obtained by administering the polypeptides or epitope-bearing fragments, analogs or cells to an animal, preferably a non human, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* Monoclonal Antibodies and Cancer Therapy, pp. 77-96, Alan R. Liss, Inc., 1985).

Techniques for the production of single chain antibodies (U.S. Patent No. 4,946,778) can also be adapted to produce single chain antibodies to polypeptides of the invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against HC polypeptides may also be employed to treat Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock., epilepsia, among others.

Another aspect of the invention relates to a method for inducing an immunological response in a mammal which comprises inoculating the mammal with HC polypeptide, or a fragment thereof, adequate to produce antibody and/or T cell immune response to protect said animal from Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others. Yet another aspect of the invention relates to a method of inducing an immunological response in a mammal which comprises, delivering a HC polypeptide via a vector directing expression of HC polynucleotide *in vivo* in order to induce such an immunological response to produce antibody to protect said animal from diseases.

A further aspect of the invention relates to an immunological/vaccine formulation (composition) which, when introduced into a mammalian host, induces an immunological response in that mammal to a HC polypeptide, wherein the composition comprises a HC polypeptide or HC gene. The vaccine formulation may further comprise a suitable carrier. Since HC polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation isotonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in-water systems and other systems known in the art. The dosage will depend on the specific activity of the vaccine and can be readily determined by routine experimentation.

HC polypeptides of the present invention may be employed in a screening process for candidate compounds which, directly or indirectly, activate (agonists) or inhibit activation of (antagonists, or otherwise called inhibitors) HC polypeptide of the present invention. Thus, the polypeptides of the invention may also be used to identify agonists or antagonists from, for example, cells, cell-free preparations, chemical libraries, and natural product mixtures. These agonists or antagonists may be natural substrates, ligands, receptors, etc, as the case may be, of the polypeptide of the present invention; or may be structural or functional mimetics of the polypeptide of the present invention. See Coligan *et al., Current Protocols in Immunology* 1(2):Chapter 5 (1991).

The choice of the candidate compounds may be based on similarity with known agonists or antagonists of carnosinase. In particular, known antagonists or agonists of the metalloprotease family may be used to determine candidate compounds to be screened.

Such known compounds include, but are not limited to, bestatine, phosphoramidon, 1,10-phenanthroline, p-hydroxymercuribenzoic acid, thiorphan.

Similarity of the candidate compounds with compounds known to activate or inhibit activation of HC may be structural or functional, or both. For example, candidate compounds to be screened may be chosen starting from their chemical resemblance to known compounds. Other candidate compounds may be chosen by taking into account their functional relationship with the metalloprotease family.

Other candidate compounds can be chosen among compounds known to be involved in a large number of biological processes and can be selected for screening solely on this basis.

HC polypeptides are thought to be responsible for many biological functions, including many pathologies relating to an abnormal expression of HC. Accordingly, it is desirous to find compounds and drugs which stimulate HC polypeptide activity or its expression (agonists) on the one hand and which can inhibit HC polypeptide activity or its expression (antagonists, inhibitors) on the other hand, for therapeutic and prophylactic purposes for such conditions as Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia. In general, such screening procedures may involve using appropriate cells which express HC polypeptide or respond to HC polypeptide of the present invention. Such cells include cells from mammals, yeast, *Drosophila* or *E. coli.* Cells which express HC polypeptide (or cell membrane containing the expressed polypeptide) or respond to HC polypeptide are then contacted with a test compound to observe binding, stimulation or inhibition of a functional response.

The assays may simply test binding of a candidate compound, wherein adherence to the cells bearing the HC polypeptide is detected by means of a label directly or indirectly associated with the candidate compound or in an assay involving competition with a labeled competitor. Further, these assays may test whether the candidate compound results in a signal generated by activation of the HC polypeptide, using detection systems appropriate to the cells bearing the HC polypeptide. Inhibitors of activation are generally assayed in the presence of a known agonist and the effect on activation by the agonist by the presence of the candidate compound is observed. Standard methods for conducting such screening assays are well understood in the art.

Examples of potential HC polypeptide antagonists include antibodies or, in some cases, oligonucleotides or proteins which are closely related to the ligands, substrates, receptors, etc, as the case may be, of the HC polypeptide, e.g., a fragment of the ligands, substrates, receptors, or small molecules which bind to the polypeptide of the present invention but do not elicit a response, so that the activity of the polypeptide is prevented.

Thus, the invention also encompasses agonists or antagonists of HC polypeptide identifiable by the methods above described.

The invention also provides methods of treating abnormal conditions related to both an over-activity or an insufficient activity of HC.

In a first aspect, the invention provides a method of treating an individual in need of an altered, diminished or inhibited level of HC activity or expression, comprising administering to such an individual a pharmaceutical composition comprising an effective amount of an active ingredient capable of altering or diminishing HC activity.

Thus this first aspect of the invention includes the use of an active ingredient capable of altering or diminishing HC activity, for the preparation of a medicament for treatment of conditions related to an over-activity of HC.

In that case, several approaches are available. One approach comprises administering as active ingredient an inhibitor compound (antagonist) identifiable by the method above described, optionally with a pharmaceutically acceptable carrier, in an amount effective to inhibit activation by blocking activity of HC, or by inhibiting a second signal, and thereby alleviating the abnormal condition.

In another approach, soluble forms of HC polypeptides still capable of binding the ligand in competition with endogenous HC may be administered as active ingredient. Typical embodiments of such competitors comprise fragments of the HC polypeptide.

In still another approach, expression of the gene encoding endogenous HC can be inhibited using expression blocking techniques. Such known techniques involve the use of antisense sequences, either internally generated or separately administered. See, for example, O'Connor, *J Neurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res.* (1979) 6:3073; Cooney *et al., Science* (1988) 241:456; Dervan *et al., Science* (1991) 251:1360. These oligomers can be administered *per se* or the relevant oligomers can be expressed *in vivo.*

In a second aspect, the invention provides a method of treating an individual in need of an increased level of HC activity or expression, comprising administering to such an individual a pharmaceutical composition comprising an effective amount of an active ingredient capable of increasing HC activity.

Thus the second aspect of the invention includes the use of an active ingredient capable of increasing HC activity, for the preparation of a medicament for treatment of conditions related to an under-activity of HC.

In that case, several approaches are also available. One approach comprises administering as active ingredient a therapeutically effective amount of a compound which activates HC, i.e., an agonist identifiable by the method described above, in combination with a pharmaceutically acceptable carrier, to thereby alleviate the abnormal condition.

Alternatively, gene therapy may be employed to effect the endogenous production of HC by the relevant cells in the subject. For example, a polynucleotide of the invention may be engineered for expression in a replication defective retroviral vector, as discussed above. The polynucleotide constitutes in that case the active ingredient. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, Gene Therapy and other Molecular Genetic-based Therapeutic Approaches, (and references cited therein) in *Human Molecular Genetics,* T Strachan and A P Read, BIOS Scientific Publishers Ltd (1996).

Pharmaceutical compositions of the invention are intended, but not limited to, the treatment and/or prevention of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.

In a first aspect, the pharmaceutical compositions of the invention for preventing and/or treating in a subject a disease or susceptibility to a disease related to over-activity or over-expression of HC may comprise a therapeutically effective amount of at least an antagonist of HC or a derivative or homologue thereof as defined above, and/or at least a nucleic acid molecule that inhibits the expression of the nucleotide sequence encoding HC (anti-sense sequence) and/or at least a peptide which competes with said receptor, as discussed above.

In a second aspect, the pharmaceutical compositions of the invention for preventing and/or treating in a subject a disease or susceptibility to a disease related to under-activity or under-expression of HC may comprise a therapeutically effective amount of at least an agonist of HC or a derivative or homologue thereof as defined above, and/or at least a polynucleotide according to the invention and/or at least a nucleotide sequence according to the invention able to express *in vivo* a HC polypeptide of the invention.

The pharmaceutical compositions in which at least a polypeptide according to the invention is used may comprise the recombinant vectors of the invention, able to express *in vivo* a polypeptide according to the invention. Polypeptides according to the invention may be also used "naked", i.e. without being inserted into a vector. It has been shown in the art that DNA or RNA sequences can be expressed in some tissues without the aid of an expression vector.

Pharmaceutical compositions according to the invention, for example those which comprise polypeptides of the invention, such as the soluble form of HC polypeptides or small molecule agonists or antagonists, may be formulated in combination with a suitable pharmaceutical carrier. Such formulations comprise a therapeutically effective amount of the polypeptide or compound, and a pharmaceutically acceptable carrier or excipient. Such carriers include but are not limited to saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, intrasternal, intraarterial or intraperitoneal, can be used. Alternative means for systemic administration include transmucosal and transdermal administration using penetrants such as bile salts or fusidic acids or other detergents. In addition, if properly formulated in enteric or encapsulated formulations, oral, rectal, intravaginal administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 1 µg/kg/day to 10 mg/kg/day of subject. More preferably, this dose is at least between 0.01 mg/kg/day and 1 mg/kg/day. Variations in the needed dosage are to be expected in view of the nature of the compounds used and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide of the invention, such as a DNA or RNA, to encode a polypeptide *ex vivo,* and for example, by the use of a retroviral plasmid vector. The cells are then introduced into the subject.

The invention further relates to the use of HC polypeptides of the invention, for the treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.

The invention also relates to the use of a HC polypeptide, for the preparation of a pharmaceutical composition for the treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.

The examples below are carried out using standard techniques, which are well known and routine to those of skill in the art, except where otherwise described in detail. The examples illustrate, but do not limit, the invention.

### Example 1

### Cloning of human carnosinase.

The sequence of HC is first identified by searching a database containing approximately 2 million human ESTs, which was generated using high throughput automated DNA sequence analysis of randomly selected human cDNA clones (Adams, M. D. *et al., Nature 377*:3-174 (1995); Adams, M. D. *et al., Nature 355*:632-634 (1992); and Adams, M. D. *et al., Science 252*:1651-1656 (1991)). Sequence homology comparisons of each EST are performed against the GenBank database using the blastn and tblastn algorithms (Altschul, S. F. *et al., J. Mol. Biol. 215*:403-410 (1990)). No homologous human sequences were identified. One homologous sequence - yeast hypothetical 52.9 KD protein - shows around 45 % similarity with HC. Electronic analysis of the distribution of the EST reveals its brain-specific localisation. Sequence of the gene is confirmed by double strand DNA sequencing and the gene is shown to be completely new by a blast search against Genbank.

### Example 2

### Cloning and Expression of HC in Mammalian Cells.

A typical mammalian expression vector contains the promoter element, which mediates the initiation of transcription of mRNA, the protein coding sequence, and signals required for the termination of transcription and polyadenylation of the transcript. Additional elements include enhancers, Kozak sequences and intervening sequences flanked by donor and acceptor sites for RNA splicing. Highly efficient transcription can be achieved with the early and late promoters from SV40, the long terminal repeats (LTRS) from Retroviruses, e.g., RSV, HTLVI, HIVI and the early promoter of the cytomegalovirus (CMV). However, cellular elements can also be used (e.g., the human actin promoter). Suitable expression vectors for use in practicing the present invention include, for example, vectors such as PSVL and PMSG (Pharmacia, Uppsala, Sweden), pRSVcat (ATCC 37152), pSV2dhfr (ATCC 37146) and pBC12Ml (ATCC 67109) and pcDNA3 (Invitrogen). Mammalian host cells that could be used include, human HEK 293, H9 and Jurkat cells, mouse NIH3T3 and C127 cells, Cos 1, Cos 7 and mouse L cells and Chinese hamster ovary (CHO) cells.

Alternatively, the gene can be expressed in stable cell lines that contain the gene integrated into a chromosome. The co-transfection with a selectable marker such as dhfr, gpt, neomycin, zeocin or hygromycin allows the identification and isolation of the transfected cells.

The transfected gene can also be amplified to express large amounts of the encoded protein. The DHFR (dihydrofolate reductase) marker is useful to develop cell lines that carry several hundred or even several thousand copies of the gene of interest. Another useful selection marker is the enzyme glutamine synthase (GS) (Murphy *et al., Biochem. J*. *227*:277-279 (1991); Bebbington *et al., Bio/Technology 10*:169-175 (1992)). Using these markers, the mammalian cells are grown in selective medium and the cells with the highest resistance are selected. These cell lines contain the amplified gene(s) integrated into a chromosome. Chinese hamster ovary (CHO) and NSO cells are often used for the production of proteins.

The expression vector pcDNA3 (Invitrogen) contains the strong promoter (CMV) of the Cytomegalovirus. Multiple cloning sites allow the cloning of foreign genes. The entire coding region of HC is inserted into the expression vector pCDNA3 (+) by use of EcoR1 and Xhol restriction enzymes. The presence of insert and its orientation in the vector is detected by PCR with specific oligonucleotides.

The expression vector carrying HC is used to transfect CHO cells, using standard protocols. Expression of HC polypeptide is detected by Western blot with anti-HC antibodies. Cell lysates and cell supernatants are analyzed.

### Example 3

### Tissue distribution of HC mRNA expression.

Northern blot analysis can be carried out to examine HC gene expression in human tissues, using methods described by, among others, Sambrook *et al.,* cited above. A cDNA probe containing the entire nucleotide sequence of the HC protein can be labeled with ³²P using the Rediprime™ DNA labeling system (Amersham Life Science, Arlington, IL), according to manufacturer's instructions. After labeling, the probe can be purified using a CHROMA SPIN - 100^{TM} column (Clontech Laboratories, Inc.), according to manufacturer's protocol number PT1200-1. The purified labeled probe is then used to examine various human tissues for HC mRNA.

### MTN blot.

Multiple Tissue Northern (MTN) blots containing various human tissues can be obtained from Clontech and examined with the labeled probe using ExpressHyb™ hybridization solution (Clontech) according to manufacturer's protocol number PT1190-1. Following hybridization and washing, the blots can be mounted and exposed to film at -70°C overnight, and films developed according to standard procedures.

### Master blot.

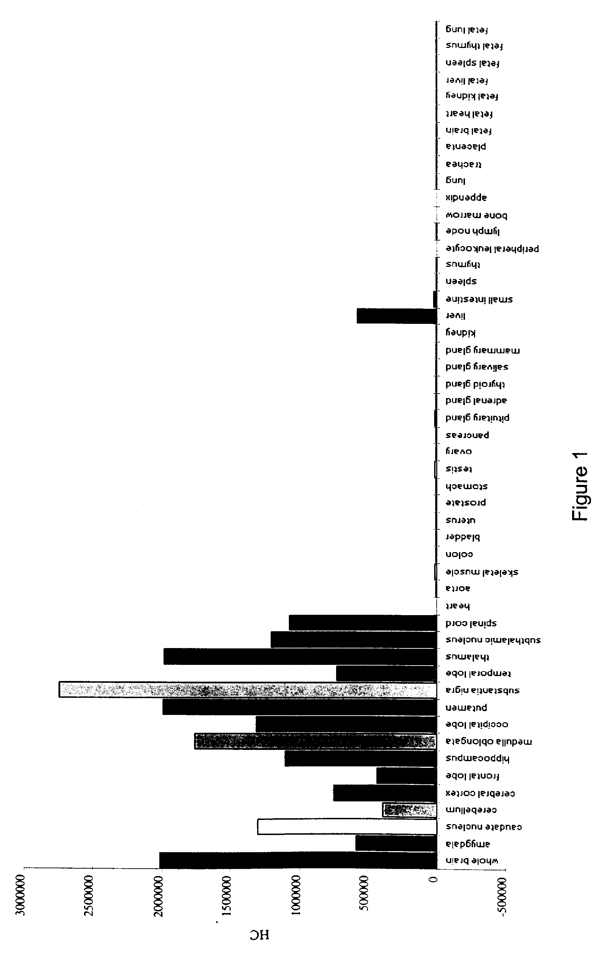

Tissue distribution of HC is determined by hybridization of the cDNA probe obtained from the HC clone by Ecorl-Xhol digestion. The probe is radiolabelled with [32P]dCTP. This probe is used to hybridize a Human RNA Master Dot blot (Clontech, ref 7770-1) with 50 different tissues represented (100-500 ng / lane) in Express Hyb buffer (Clontech) according to the manufacturer specifications. The signal is detected using a Storm (Molecular Dynamics). Results show that HC mRNA is only expressed in CNS and liver (Figure 1).

### Example 4

### Functional activity of Human Carnosinase by in vitro measurements.

Experiments are performed on transiently transfected CHO cells obtained using standard techniques. Two protocols are applied : 1) tissue carnosinase, and 2) plasma carnosinase. Both protocols give comparable results when applied to transiently transfected cells.

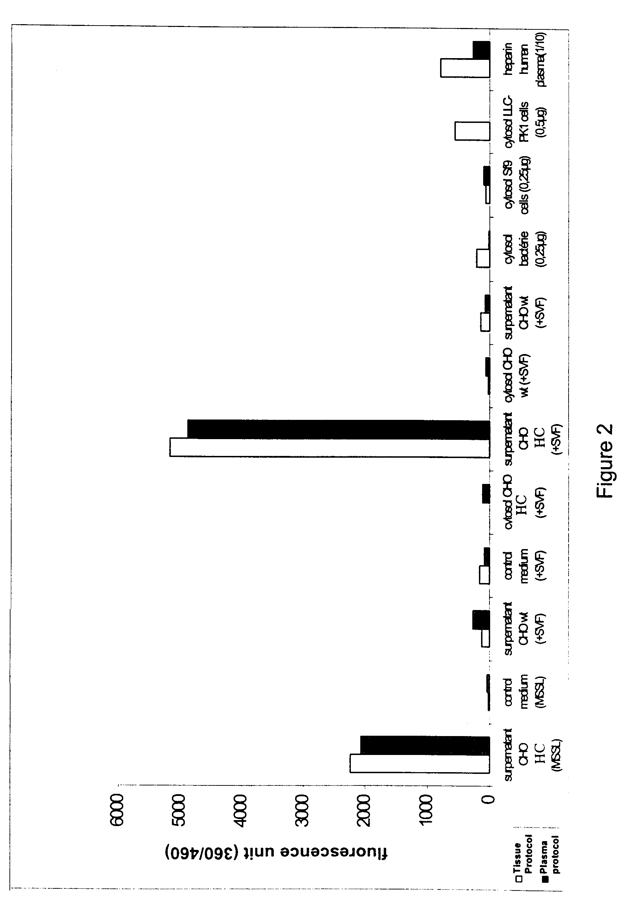

Cells transfected with HC cDNA are diluted in 10mM Tris-HCI pH 8.0. Cytosol is used for activity measurement in presence or absence of 0.25 mM MnCl₂, sonicated 3 times 10 sec. on ice and centrifuged 15,000 g / 10 min. at 4°C. Enzymatic assay is performed in black 96-well plates. 5 µl of sample are mixed with 5 µl of buffer A (125 mM glycine pH 9.5; 0.25 mM MnCl₂ in 10 mM Tris-HCI pH 8.0, 10 mM dithiothreitol) and incubated at room temperature for 15 min. 5 µl of substate (50 mM carnosine in 125 mM glycine, pH 9.5) are added and incubated at 30°C for 60 min. The reaction is stopped by precipitation with 15 µl of 0.6N TCA. Measurement of Histidine is performed after precipitation with 30 µl of supernatant by addition of 100 µl of 1N NaOH and 50 µl of OPT (50 mg o-phthalaldehyde / 100 ml ethanol 95%), incubation at 30°C for 15 min., addition of 50 µl of buffer B (56.36 g P₂O₇ K₄.10H₂O + 1.65 g HNa₂O₃S/1 liter 8N phosphoric acid), incubation at 30°C for 15 min. Finally, the plate is agitated and reading of the fluorescence is performed at room temperature (excitation 360 nm, emission 460 nm). Results appear on Figure 2. Carnosinase activity is found exclusively in the medium of transfected cells. Two protocols are tested with cells grown in two different mediums : serum-free (MSSL) medium or medium supplied with fetal serum (+SVF). In both conditions, high activity of carnosinase is found.

### Example 5.

### Expression of Human Carnosinase in Alzheimer's disease.

Analysis of the level of expression of HC in patients with Alzheimer's disease is performed by Northern blot and quantitative PCR using standard protocols. Commercially available blots with total RNAs from different cerebral structures of Alzheimer and control patients (Invitrogen) are hybridized with HC cDNA probe. For quantitative PCR mRNAs are isolated from temporal cortex of control and Alzheimer patients. After the reaction of reverse transcription, quantitative PCR is done using commercially available kit from Clontech. Results demonstrate a 2.5 fold increase in mRNA level for HC in patients with Alzheimer's disease.

It will be clear that the invention may be practiced otherwise than as particularly described in the foregoing description and examples.

Numerous modifications and variations of the present invention are possible in light of the above teachings and, therefore, are within the scope of the appended claims.

The entire disclosure of all publications (including patents, patent applications, journal articles, laboratory manuals, books, or other documents) cited herein are incorporated by reference.

## Claims

1. An isolated polynucleotide comprising a nucleotide sequence at least 80% identical to a nucleotide sequence selected from the group consisting of :
(a) the nucleotide sequence set forth in SEQ ID NO: 1;
(b) a nucleotide sequence encoding the HC polypeptide set forth in SEQ ID NO:2;
(c) the nucleotide sequence comprising the sequence located between nucleotide numbers 1 to 1524 set forth in SEQ ID NO: 1;
(d) the nucleotide sequence encoding a polypeptide comprising amino acids from about 255 to about 272 set forth in SEQ ID NO:2;
(e) the nucleotide sequence encoding a polypeptide comprising amino acids from about 475 to about 490 set forth in SEQ ID NO:2;
(f) a nucleotide sequence encoding the HC polypeptide expressed by the cDNA clone contained in ATCC deposit number PTA-541;
(g) a nucleotide sequence able to hybridize with the nucleotide sequence set forth in SEQ ID NO:1 or the cDNA clone contained in ATCC deposit number PTA-541;
(h) a nucleotide sequence encoding the mature HC polypeptide expressed by the cDNA clone contained in ATCC deposit number PTA-541;
(i) a nucleotide sequence complementary to any of the nucleotide sequences defined in (a), (b), (c), (d), (e), (f), (g) or (h) above, as well as fragments, derivatives, variants, mutated forms of these nucleotide sequences.

2. Polynucleotide according to claim 1, wherein it comprises the nucleotide sequence located between nucleotide number 1 and nucleotide number 500, or the nucleotide sequence located between nucleotide number 501 and nucleotide number 1524 of the sequence set forth in SEQ ID NO:1.

3. A DNA or RNA molecule constituting a recombinant vector, comprising an expression system which is capable of producing a HC polypeptide when said expression system is present in a compatible host, wherein said DNA or RNA molecule comprises a polynucleotide according to claim 1 or 2.

4. A host cell comprising the expression system of claim 3.

5. A process for producing a HC polypeptide comprising the steps of transforming or transfecting a host cell with an expression vector according to claim 3, culturing said host cell under appropriate conditions so that said host cell produces HC polypeptide, and recovering the polypeptide from the culture.

6. An isolated HC polypeptide comprising an amino acid sequence which is at least 80% identical to a sequence selected from the group consisting of :
(a) the amino acid sequence of SEQ ID NO:2;
(b) the amino acid sequence of HC polypeptide having the complete amino acid sequence encoded by the cDNA clone contained in ATCC deposit number PTA-541;
(c) the amino acid sequence of the mature HC polypeptide having the complete amino acid sequence encoded by the cDNA clone contained in ATCC deposit number PTA-541; as well as fragments, derivatives, variants or mutated forms of these amino acid sequences.

7. Polypeptide according to claim 6, susceptible to be obtained by the process of claim 5 or by chemical synthesis.

8. An antibody, or a fragment thereof, able to recognize a HC polypeptide according to claim 6.

9. Antibody according to claim 8 able to recognize a polypeptide comprising amino acid residues from about 255 to about 272 of SEQ ID NO:2, a polypeptide comprising amino acid residues from about 475 to about 490 of SEQ ID NO:2.

10. A method for identifying agonists of HC polypeptide comprising the steps of:
(a) contacting a host cell according to claim 4 with a candidate compound; and
(b) determining whether the candidate compound effects a signal generated by activation of HC polypeptide.

11. An agonist of HC polypeptide identifiable by the method of claim 10.

12. An agonist according to claim 11, wherein the candidate compound is selected among compounds which present a structural or functional, or both, similarity with known agonists of carnosinase or of the metalloprotease family, or compounds which are known to be involved in a large number of biological processes.

13. A method for identifying antagonists of HC polypeptide comprising the steps of :
(a) contacting a host cell according to claim 4 with an agonist of HC polypeptide, and
(b) determining whether the signal generated by said agonist is diminished in the presence of a candidate compound.

14. An antagonist of HC polypeptide identifiable by the method of claim 13.

15. Antagonist according to claim 14, wherein the candidate compound is selected among compounds which present a structural or functional, or both, similarity with known antagonists of carnosinase or of the metalloprotease family, or compounds which are known to be involved in a large number of biological processes.

16. A process for diagnosing a disease or a susceptibility to a disease in a subject related to expression or activity of a HC polypeptide according to claim 6 in a subject comprising:
(a) determining the presence or absence of a mutation in the nucleotide sequence encoding said HC polypeptide in the genome of said subject; and/or
(b) analyzing for the presence or amount of said HC polypeptide expression in a sample derived from said subject.

17. Pharmaceutical composition, for preventing and/or treating in a subject a disease or susceptibility to a disease related to an under-expression or under-activity of HC polypeptide, said composition comprising a therapeutically effective amount of at least an agonist of HC polypeptide or a derivative or homologue thereof identified by the method according to claim 10, and/or at least a HC polypeptide according to claim 6, and/or at least a nucleotide sequence able to express *in vivo* a HC polypeptide according to claim 6.

18. Pharmaceutical composition for preventing and/or treating in a subject a disease or susceptibility to a disease related to over-activity or over-expression of HC polypeptide, said composition comprising a therapeutically effective amount of at least an antagonist of HC polypeptide or a derivative or homologue thereof identified by the method according to claim 13, and/or at least a nucleic acid molecule that inhibits the expression of the nucleotide sequence encoding HC polypeptide and/or at least a peptide which competes with HC polypeptide.

19. Pharmaceutical composition according to claim 17 or 18, comprising a recombinant vector according to claim 3, able to express *in vivo* a polypeptide according to claim 6.

20. Pharmaceutical composition according to any of claims 17 to 19, further comprising at least a pharmaceutically acceptable carrier.

21. Pharmaceutical composition according to any of claims 17 to 20 for prevention and/or treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.

22. Use of a HC polypeptide according to claim 6, for the treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.

23. Use of a HC polypeptide according to claim 6, for the preparation of a pharmaceutical composition for the treatment of Alzheimer's disease and cognitive disorders, developmental abnormalities and fetal deficiencies, neurodegenerative disorders such as amyotrophic lateral sclerosis, Parkinson's disease, schizophrenia, abnormal mental states, ischemic shock, epilepsia, among others.
